# FASCICULE DE BREVET EUROPEEN

(11) **EP 1 280 458 B1**
(45) Date de publication et mention de la délivrance du brevet: **06.10.2004**
(21) Numéro de dépôt: 01929761.3
(22) Date de dépôt: 27.04.2001
(51) Int. Cl.: A61B 5/15, G01N 1/00, G01N 33/49

(54) **DISPOSITIF DE PRELEVEMENT A TUBULURES DE SECTION DIFFERENTE**
ENTNAHMEVORRICHTUNG MIT RÖHRCHEN UNTERSCHIEDLICHEN QUERSCHNITTS
SAMPLING DEVICE WITH TUBES HAVING DIFFERENT CROSS-SECTION

(30) Priorité: 12.05.2000 FR 0006081
(43) Date de publication de la demande: 05.02.2003
(73) Titulaire: MACO PHARMA, 59420 Mouvaux (FR)
(72) Inventeur: VERPOORT, Thierry, F-59420 Mouvaux (FR); GOUDALIEZ, Francis, F-59155 Faches Thumesnil (FR)
(74) Mandataire: Geismar, Thierry
(86) Numéro de dépôt international: PCT/FR2001/001329
(87) Numéro de publication internationale: WO 2001/085029

(56) Documents cités:
- EP-A- 0 154 002
- EP-A- 0 256 415
- US-A- 4 223 672

## Description

L'invention est relative à un dispositif de prélèvement d'un fluide biologique auquel une solution anticoagulante et/ou de conservation doit être additionnée.

Elle s'applique typiquement au cas où du sang total est prélevé d'un donneur pour être collecté de façon stérile dans une poche de recueil.

Pour empêcher la coagulation du sang dans la poche de recueil, il est classique que la poche de recueil soit remplie préalablement au prélèvement avec une solution anticoagulante et/ou de conservation.

Un des problèmes qui se pose est alors de réaliser, notamment par agitation de la poche de recueil, un mélange homogène entre la solution contenue dans la poche et le sang prélevé.

Un autre problème qui se pose est le contrôle de la proportion volumique de solution dans le fluide prélevé.

En effet, notamment dans le domaine du prélèvement sanguin, la quantité de solution anticoagulante et/ou de conservation présente dans une poche de sang est fixée à une certaine valeur pour que le sang soit utilisable dans le domaine médical.

Pour résoudre ces problèmes, des dispositifs comprenant une poche contenant la solution anticoagulante et/ou de conservation du fluide prélevé et une poche de recueil initialement vide ont été proposés.

Dans de tels dispositifs, des pompes présentant deux rotors, l'un pour l'alimentation en fluide et l'autre pour l'alimentation en solution, sont utilisées. Lors de l'alimentation, les deux rotors tournent à des vitesses tangentielles différentes et asservies l'une par rapport à l'autre de sorte à obtenir la proportion volumique voulue.

Ces dispositifs ne donnent pas entièrement satisfaction en ce qu'ils nécessitent l'utilisation d'une structure de pompe complexe et particulière qui complique leur utilisation en augmentant le coût du prélèvement.

Par ailleurs, on connaît du document US-4 223 672 un appareil de traitement extracorporel du sang dans lequel un récipient contenant un anticoagulant est connecté à une première tubulure par l'intermédiaire d'une quatrième tubulure. Cette connexion est réalisée en amont d'une pompe péristaltique qui est prévue pour alimenter un récipient par l'intermédiaire de ladite première tubulure. Ce document indique que le rapport entre les sections des première et quatrième tubulures est choisi pour obtenir le mélange voulu d'anticoagulant dans le sang.

Dans ce type d'appareils, du fait que la connexion est réalisée en amont de la pompe, le flux de solution anticoagulante est dirigé vers le patient. Cette réalisation présente donc notamment l'inconvénient d'avoir un risque d'injection de solution anticoagulante au patient.

L'invention vise donc à remédier à ces inconvénients en proposant un dispositif de prélèvement permettant de contrôler la proportion volumique de solution additionnée au fluide prélevé en utilisant une pompe péristaltique de structure classique, ledit dispositif évitant toute injection accidentelle de solution additionnée au patient.

A cet effet, l'invention propose un dispositif de prélèvement d'un fluide biologique, notamment sanguin, du type comprenant des moyens de prélèvement dudit fluide, au moins une poche contenant une solution anticoagulante et/ou de conservation du fluide prélevé, et au moins une poche de recueil destinée à recevoir le fluide prélevé additionné de la solution anticoagulante et/ou de conservation, dans lequel la poche de recueil est en communication fluidique avec les moyens de prélèvement par l'intermédiaire d'au moins une première tubulure souple et avec ladite poche par l'intermédiaire d'au moins une deuxième tubulure souple, ledit dispositif comprenant en outre une pompe péristaltique apte à écraser partiellement respectivement la première et la deuxième tubulure sur une zone dite d'écrasement, dans lequel d'une part la vitesse d'écrasement de chaque tubulure est sensiblement identique et d'autre part le rapport entre la section de la deuxième tubulure traversée par la solution et celle de la première tubulure traversée par le fluide est sensiblement égal à la proportion volumique de solution anticoagulante et/ou de conservation à additionner au fluide prélevé, de sorte que lorsque la pompe est actionnée la poche de recueil est alimentée avec le fluide et avec la solution anticoagulante et/ou de conservation dans la proportion volumique voulue, et la première et la deuxième tubulures sont agencées pour permettre le mélange de la solution anticoagulante et/ou de conservation avec le fluide en aval de la zone d'écrasement.

La pompe péristaltique est apte à écraser la première et la deuxième tubulure simultanément de sorte à alimenter la poche de recueil avec le fluide et avec la solution anticoagulante et/ou de conservation de façon simultanée.

En variante, une jonction est prévue en aval de la zone d'écrasement de sorte à connecter la première et la deuxième tubulure à une première extrémité d'une tubulure dont la deuxième extrémité est connectée à un port d'entrée de la poche de recueil. La pompe péristaltique comprend alors un moteur actionnant un arbre sur lequel sont montés deux rotors cylindriques identiques, lesdits rotors portant chacun des galets aptes à écraser respectivement la première et la deuxième tubulure.

Suivant une réalisation, en amont de la zone d'écrasement, la première et la deuxième tubulure sont associées de façon étanche dans une tubulure dont l'extrémité aval est connectée à un port d'entrée de la poche de recueil.

En variante, en aval de la zone d'écrasement, la première et la deuxième tubulure sont interrompues de sorte que la poche de recueil soit alimentée en fluide et en solution anticoagulante et/ou de conservation par l'intermédiaire de la tubulure.

Suivant une autre réalisation, en amont de la zone d'écrasement, la seconde tubulure est introduite de façon étanche à l'intérieur de la première tubulure.

En variante, en aval de la zone d'écrasement, la deuxième tubulure est interrompue de sorte que la poche de recueil soit alimentée en fluide et en solution anticoagulante et/ou de conservation par l'intermédiaire de la première tubulure.

Suivant ces deux réalisations, la pompe péristaltique comprend un moteur actionnant un arbre sur lequel est monté un rotor cylindrique, ledit rotor portant des galets aptes à écraser la première et la deuxième tubulure.

Dans un exemple particulier, le rapport entre la section de la deuxième tubulure traversée par la solution et celle de la première tubulure traversée par le fluide est compris entre 0,05 et 0,5, notamment égal à 0,14.

D'autres objets et avantages de l'invention apparaîtront au cours de la description qui suit en référence aux dessins annexés.

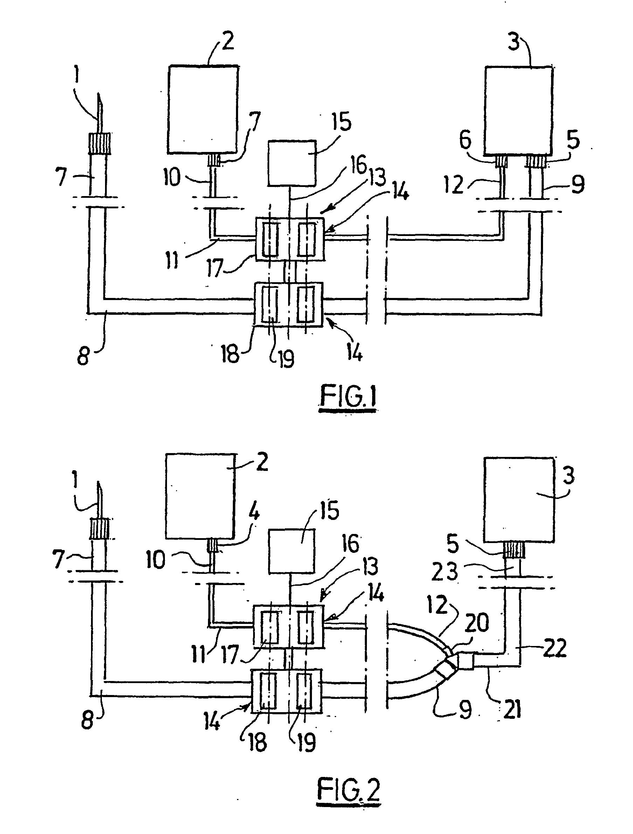

La figure 1 représente schématiquement, en élévation et à plat, une première variante d'un premier mode de réalisation du dispositif de prélèvement dans laquelle la poche de recueil est alimentée directement par la première et par la deuxième tubulure, lesdites tubulures étant écrasées chacune par un rotor différent.

La figure 2 représente schématiquement, en élévation et à plat, une deuxième variante du premier mode de réalisation dans laquelle une jonction est prévue en aval de la zone d'écrasement de sorte à alimenter la poche de recueil avec une tubulure unique.

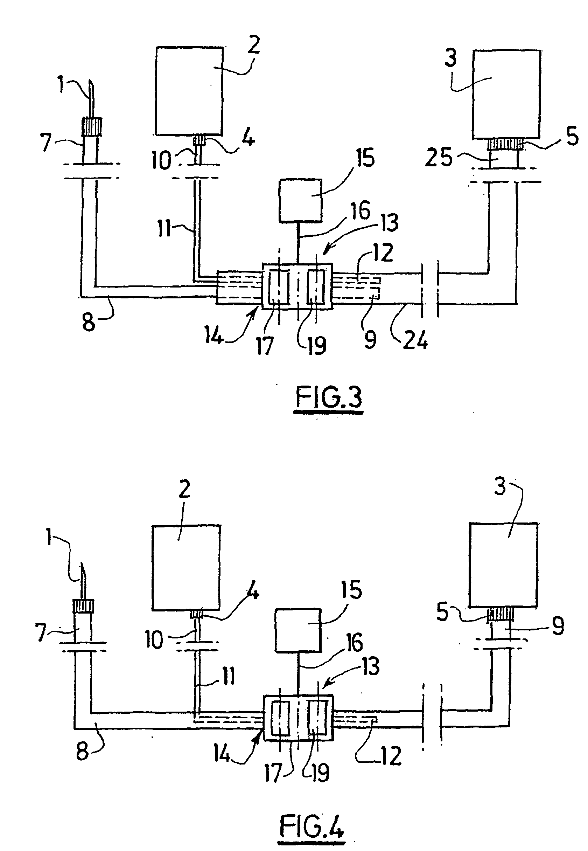

La figure 3 représente schématiquement, en élévation et à plat, un deuxième mode de réalisation du dispositif de prélèvement dans lequel la poche de recueil est alimentée par une tubulure unique dans laquelle sont associées la première et la deuxième tubulure, lesdites tubulures étant écrasées par un même rotor.

La figure 4 représente schématiquement, en élévation et à plat, un troisième mode de réalisation du dispositif de prélèvement dans lequel la poche de recueil est alimentée par la première tubulure dans laquelle la deuxième tubulure est disposée, lesdites tubulures étant écrasées par un même rotor.

Dans les réalisations particulières représentées sur les figures, un dispositif de prélèvement d'un fluide biologique comprend des moyens de prélèvement 1 dudit fluide, une poche 2 contenant une solution anticoagulante et/ou de conservation du fluide prélevé et une poche de recueil 3 destinée à recevoir le fluide prélevé additionné de la solution anticoagulante et/ou de conservation. Un tel dispositif est notamment destiné à collecter du sang total prélevé d'un donneur à l'aide des moyens de prélèvement 1, par exemple formés d'une aiguille. La solution anticoagulante et/ou de conservation est par exemple de type CPD et est introduite dans la poche 2 préalablement au prélèvement.

La poche de recueil 3 et la poche 2 contenant la solution ont par exemple une structure analogue comprenant une enveloppe extérieure formée de deux feuilles de matière plastique assemblées, par exemple par soudage, sur leur périphérie de sorte à définir un volume intérieur destiné à recevoir le contenu.

Les feuilles sont formées en matériau plastique souple biocompatible soudable et stérilisable, par exemple en polychlorure de vinyle.

L'enveloppe extérieure de chacune des poches 2, 3 est munie d'au moins un orifice 4, 5, 6 agencé pour permettre l'alimentation du volume intérieur et/ou la distribution du contenu de la poche 2, 3.

Les moyens de prélèvement 1 sont associés à une extrémité 7 d'une première tubulure souple 8 dont l'autre extrémité 9 est en communication fluidique avec la poche de recueil 3. Cette réalisation permet l'alimentation, en circuit fermé, de la poche de recueil 3 en fluide prélevé.

La poche 2 contenant la solution anticoagulante et/ou de conservation est associée, par l'intermédiaire d'un orifice de sortie 4, à une extrémité 10 d'une deuxième tubulure souple 11 dont l'autre extrémité 12 est en communication fluidique avec la poche de recueil 3. Cette réalisation permet l'alimentation, en circuit fermé, de la poche de recueil 3 en solution anticoagulante et/ou de conservation.

Les tubulures 8, 11 sont par exemple formées en matériau plastique souple biocompatible stérilisable, sécable et soudable, par exemple en polychlorure de vinyle.

Le dispositif de prélèvement suivant l'invention comprend en outre une pompe péristaltique 13 apte à écraser partiellement respectivement la première 8, et la deuxième 11 tubulure sur une zone 14 dite d'écrasement.

La pompe péristaltique 13 utilisée est de type classique, à savoir comprenant un moteur 15 actionnant un arbre 16 sur lequel sont montés un ou plusieurs rotors cylindriques 17, 18 portant des galets 19. Dans ce type de pompe 13, chaque rotor 17, 18 est apte à écraser une tubulure souple disposée sur le chemin de roulement des galets 19. La succession de compression et de relaxation ainsi produite sur la tubulure souple entraîne la circulation du fluide à l'intérieur de ladite tubulure. La structure de ce type de pompe étant bien connue de l'homme du métier, elle ne sera pas décrite plus avant dans cette description.

Suivant l'invention, la vitesse d'écrasement de la première 8 et de la deuxième 11 tubulure est sensiblement identique. Dans un mode de réalisation, cette caractéristique est réalisée en utilisant un rotor 17 unique pour écraser les deux tubulures 8, 11. Dans un autre mode de réalisation, deux rotors 17, 18 sont prévus pour écraser respectivement les deux tubulures 8, 11 à une vitesse tangentielle identique lors de la rotation de l'arbre 16. En variante, ces deux rotors 17, 18 sont identiques. Ces deux modes de réalisation sont décrits plus en détail par la suite.

Suivant une autre caractéristique de l'invention, le rapport entre la section de la deuxième tubulure 11 traversée par la solution et celle de la première tubulure 8 traversée par le fluide est sensiblement égal à la proportion volumique de solution anticoagulante et/ou de conservation à additionner au fluide prélevé.

Lorsque la pompe 13 est actionnée, les galets 19 écrasent chaque tubulure 8, 11 avec une vitesse tangentielle sensiblement identique de sorte que le débit à l'intérieur de chaque tubulure 8, 11 est proportionnel à la section traversée par le fluide. En effet, le débit à l'intérieur d'une tubulure est le produit entre la vitesse d'écoulement du fluide, c'est-à-dire ici la vitesse tangentielle d'écrasement de la tubulure, et la section de la tubulure traversée par le fluide.

Suivant l'invention, la poche de recueil 3 est ainsi alimentée avec le fluide et avec la solution anticoagulante et/ou de conservation dans la proportion volumique voulue.

On décrit ci-dessous un premier, un deuxième et un troisième mode de réalisation d'un dispositif de prélèvement dans lesquels la première 8 et la deuxième 11 tubulures sont agencés pour permettre le mélange de la solution anticoagulante et/ou de conservation avec le fluide en aval de la zone d'écrasement.

Ainsi, la solution anticoagulante et/ou de conservation n'est jamais dirigée vers le patient, ce qui évite toute injection accidentelle de cette solution au patient.

Dans la description, les termes « amont » et « aval » sont définis respectivement par rapport au sens de circulation du fluide depuis les moyens de prélèvement 1 jusqu'à la poche de recueil 3.

Dans le premier mode de réalisation (voir figures 1 et 2), la pompe péristaltique 13 comprend deux rotors cylindriques 17, 18 identiques, lesdits rotors 17, 18 portant chacun des galets 19 aptes à écraser respectivement la première 8 et la deuxième 11 tubulure.

Dans une première variante (figure 1), la première 8 et la deuxième 11 tubulure sont associées à la poche de recueil 3 respectivement par l'intermédiaire d'un orifice d'entrée 5, 6.

Dans une deuxième variante (figure 2), une jonction 20, par exemple en Y, est prévue en aval de la zone d'écrasement 14 de sorte à connecter la première 8 et la deuxième 11 tubulure à une première extrémité 21 d'une tubulure 22. La deuxième extrémité 23 de ladite tubulure 22 est connectée à un port d'entrée 5 de la poche de recueil 3. La section de la tubulure 22 doit être suffisante pour ne pas provoquer de surpression dans le dispositif, mais n'est pas fixée à une valeur précise. Dans cette variante la poche de recueil 3 est alimentée en fluide prélevé et en solution anticoagulante et/ou de conservation par l'intermédiaire de la tubulure 22.

Dans un deuxième mode de réalisation (figure 3), la première 8 et la deuxième 11 tubulure sont associées de façon étanche dans une tubulure 24 dont l'extrémité aval 25 est connectée à un port d'entrée 5 de la poche de recueil 3. Dans cette réalisation les tubulures 8, 11, 24 forment un ensemble de type à doubles lumières parallèles.

Cette association est réalisée en amont de la zone d'écrasement 14 de la pompe péristaltique 13 qui comprend un seul rotor cylindrique 17 portant des galets 19 aptes à écraser les tubulures 8, 11, 24.

En variante, la première 8 et la deuxième 11 tubulure sont interrompues en aval de la zone d'écrasement 14. Le fluide prélevé et la solution anticoagulante et/ou de conservation sont alors transportés par la tubulure 24 depuis l'extrémité aval 9, 12 des première 8 et deuxième 11 tubulures jusqu'à la poche de recueil 3.

Dans un troisième mode de réalisation (figure 4), la seconde tubulure 11 est introduite de façon étanche à l'intérieur de la première tubulure 8 de sorte à former un ensemble de type à doubles lumières concentriques. L'extrémité aval 9 de la première tubulure 8 est alors connectée à un port d'entrée 5 de la poche de recueil 3.

Dans ce mode de réalisation, la section de la première tubulure 8 traversée par le fluide est égale à la section de la première tubulure 8 moins celle de la deuxième tubulure 11.

Cette introduction est réalisée en amont de la zone d'écrasement 14 de la pompe péristaltique 13 qui comprend un seul rotor cylindrique 17 portant des galets 19 aptes à écraser la première 8 et la deuxième 11 tubulure.

En variante, la deuxième tubulure 11 est interrompue en aval de la zone d'écrasement 14. Le fluide prélevé et la solution anticoagulante et/ou de conservation sont alors transportés par la première tubulure 8 depuis l'extrémité aval 12 de la deuxième tubulure 11 jusqu'à la poche de recueil 3.

Suivant l'invention, la poche de recueil 3 peut être incorporée, par l'intermédiaire d'un orifice de sortie, dans un système plus complexe qui comprend par exemple d'autres poches, des tubulures, des clamps ou des filtres. Un tel système peut permettre, après le recueil du sang total et éventuellement élimination des moyens de prélèvement 1 et de la poche 2 par coupure et soudure des tubulures correspondantes 8, 11, 22, 24, de réaliser en circuit clos la filtration et la séparation du sang total en ses différents constituants.

Dans un exemple particulier, le dispositif est destiné à réaliser en circuit clos le prélèvement du sang total d'un donneur et l'addition d'une solution anticoagulante de type CPD.

D'après la norme en vigueur en France, la proportion volumique de solution de type CPD à ajouter au sang total est de 14%. Suivant l'invention, le rapport entre la section de la deuxième tubulure 11 traversée par la solution et celle de la première tubulure 8 traversée par le fluide est alors fixé à 0,14. Par exemple la section de la première tubulure 8 est égale à 0,7 cm² et celle de la deuxième tubulure 11 à 0,1 cm².

Dans une réalisation, la pompe péristaltique 13 est apte à écraser la première 8 et la deuxième 11 tubulure simultanément. Une telle réalisation permet d'alimenter la poche de recueil 3 avec le fluide et avec la solution anticoagulante et/ou de conservation de façon simultanée de sorte à améliorer la réalisation du mélange par agitation de la poche de recueil 3.

L'invention permet donc de réaliser le prélèvement en circuit clos d'un fluide et l'addition d'une solution anticoagulante et/ou de conservation dans une poche de recueil 3 en ayant, à tout instant, une proportion de solution dans le fluide prélevé qui est fixée à la valeur souhaitée.

Ainsi, quelle que soit la quantité de fluide prélevé, le contenu de la poche de recueil 3 est alors proportionnellement identique de sorte qu'il pourra être utilisé.

Ces caractéristiques sont obtenues en utilisant une pompe péristaltique 13 de structure classique dont le ou les rotors 17, 18 tournent à une vitesse tangentielle identique et en jouant sur la différence de section traversée par le fluide de chaque tubulure 8, 11.

## Revendications

1. Dispositif de prélèvement d'un fluide biologique, notamment sanguin, du type comprenant des moyens de prélèvement (1) dudit fluide, au moins une poche (2) contenant une solution anticoagulante et/ou de conservation du fluide prélevé, et au moins une poche de recueil (3) destinée à recevoir le fluide prélevé additionné de la solution anticoagulante et/ou de conservation, dans lequel la poche de recueil (3) est en communication fluidique avec les moyens de prélèvement (1) par l'intermédiaire d'au moins une première tubulure souple (8) et avec ladite poche (2) par l'intermédiaire d'au moins une deuxième tubulure souple (11), ledit dispositif comprenant en outre une pompe péristaltique (13) apte à écraser partiellement respectivement la première (8) et la deuxième (11) tubulure sur une zone (14) dite d'écrasement, la vitesse d'écrasement de chaque tubulure (8, 11) étant sensiblement identique et le rapport entre la section de la deuxième tubulure (11) traversée par la solution et celle de la première tubulure (8) traversée par le fluide est sensiblement égal à la proportion volumique de solution anticoagulante et/ou de conservation à additionner au fluide prélevé, de sorte que lorsque la pompe (13) est actionnée la poche de recueil (3) est alimentée avec le fluide et avec la solution anticoagulante et/ou de conservation dans la proportion volumique voulue, ledit dispositif étant **caractérisé en ce que** la première (8) et la deuxième (11) tubulures sont agencées pour permettre le mélange de la solution anticoagulante et/ou de conservation avec le fluide en aval de la zone d'écrasement (14).

2. Dispositif selon la revendication 1, **caractérisé en ce que** la pompe péristaltique (13) est apte à écraser la première (8) et la deuxième (11) tubulure simultanément de sorte à alimenter la poche de recueil (3) avec le fluide et avec la solution anticoagulante et/ou de conservation de façon simultanée.

3. Dispositif selon la revendication 1 ou 2, **caractérisé en ce qu'**une jonction (20) est prévue en aval de la zone d'écrasement (14) de sorte à connecter la première (8) et la deuxième (11) tubulure à une première extrémité (21) d'une tubulure (22) dont la deuxième extrémité (23) est connectée à un port d'entrée (5) de la poche de recueil (3).

4. Dispositif selon l'une quelconque des revendications 1 à 3, **caractérisé en ce que** la pompe péristaltique (13) comprend un moteur (15) actionnant un arbre (16) sur lequel sont montés deux rotors cylindriques (17, 18) identiques, lesdits rotors portant chacun des galets (19) aptes à écraser respectivement la première (8) et la deuxième (11) tubulure.

5. Dispositif selon la revendication 1 ou 2, **caractérisé en ce que**, en amont de la zone d'écrasement (14), la première (8) et la deuxième (11) tubulure sont associées de façon étanche dans une tubulure (24) dont l'extrémité aval (25) est connectée à un port d'entrée (5) de la poche de recueil (3).

6. Dispositif selon la revendication 5, **caractérisé en ce que**, en aval de la zone d'écrasement (14), la première (8) et la deuxième (11) tubulure sont interrompues de sorte que la poche de recueil (3) soit alimentée en fluide et en solution anticoagulante et/ou de conservation par l'intermédiaire de la tubulure (24).

7. Dispositif selon la revendication 1 ou 2, **caractérisé en ce que**, en amont de la zone d'écrasement (14), la seconde tubulure (11) est introduite de façon étanche à l'intérieur de la première tubulure (8).

8. Dispositif selon la revendication 7, **caractérisé en ce que**, en aval de la zone d'écrasement (14), la deuxième tubulure (11) est interrompue de sorte que la poche de recueil (3) soit alimentée en fluide et en solution anticoagulante et/ou de conservation par l'intermédiaire de la première tubulure (8).

9. Dispositif selon l'une quelconque des revendications 5 à 8, **caractérisé en ce que** la pompe péristaltique (13) comprend un moteur (15) actionnant un arbre (16) sur lequel est monté un rotor cylindrique (17), ledit rotor portant des galets (19) aptes à écraser la première (8) et la deuxième (11) tubulure.

10. Dispositif selon l'une quelconque des revendications 1 à 9, **caractérisé en ce que** le rapport entre la section de la deuxième tubulure (11) traversée par la solution et celle de la première tubulure (8) traversée par le fluide est compris entre 0,05 et 0,5, notamment égal à 0,14.

## Claims

1. A device for sampling a biological fluid, in particular blood, of the type comprising means (1) of sampling the said fluid, at least one bag (2) containing an anticoagulant and/or preservation solution for the fluid sampled, and at least one collecting bag (3) intended to receive the sampled fluid with the anticoagulant and/or preservation solution added, in which the collecting bag (3) is in fluid communication with the sampling means (1) through at least one first flexible tube (8) and with the said bag (2) through at least one second flexible tube (11), the said device also comprising a peristaltic pump (13) able to partially squash respectively the first (8) and second (11) tubes on a so-called squashing zone (14), the speed of squashing of each tube (8, 11) being substantially identical and the ratio between the cross-section of the second tube (11) through which the solution passes and that of the first tube (8) through which the fluid passes is substantially equal to the proportion by volume of anticoagulant and/or preservation solution to be added to the fluid sampled, so that, when the pump (13) is actuated, the collecting bag (3) is supplied with the fluid and with the anticoagulant and/or preservation solution in the required proportion by volume, the said device being **characterised in that** the first (8) and second (11) tubes are arranged so as to allow the mixing of the anticoagulant and/or preservation solution with the fluid downstream of the squashing zone (14).

2. A device according to Claim 1, **characterised in that** the peristaltic pump (13) is able to squash the first (8) and second (11) tubes simultaneously so as to supply the collecting bag (3) with the fluid and with the anticoagulant and/or preservation solution simultaneously.

3. A device according to Claim 1 or 2, **characterised in that** a junction (20) is provided downstream of the squashing zone (14) so as to connect the first (8) and second (11) tubes to a first end (21) of a tube (22) whose second end (23) is connected to an inlet portion (5) of the collecting bag (3).

4. A device according to any one of Claims 1 to 3, **characterised in that** the peristaltic pump (13) comprises a motor (15) actuating a shaft (16) on which two identical cylindrical rotors (17, 18) are mounted, the said rotors each carrying rollers (19) able to squash respectively the first (8) and second (11) tubes.

5. A device according to Claim 1 or 2, **characterised in that**, upstream of the squashing zone (14), the first (8) and second (11) tubes are associated sealingly in a tube (24) whose downstream end (25) is connected to an inlet port (5) of the collecting bag (3).

6. A device according to Claim 5, **characterised in that**, downstream of the squashing zone (14), the first (8) and second (11) tubes are interrupted so that the collecting bag (3) is supplied with fluid and anticoagulant and/or preservation solution by means of the tube (24).

7. A device according to Claim 1 or 2, **characterised in that**, upstream of the squashing zone (14), the second tube (11) is introduced sealingly inside the first tube (8).

8. A device according to Claim 7, **characterised in that**, downstream of the squashing zone (14), the second tube (11) is interrupted so that the collecting bag (3) is supplied with fluid and anticoagulant and/or preservation solution by means of the first tube (8).

9. A device according to any one of Claims 5 to 8, **characterised in that** the peristaltic pump (13) comprises a motor (15) actuating a shaft (16) on which a cylindrical rotor (17) is mounted, the said rotor carrying rollers (19) able to squash the first (8) and second (11) tubes.

10. A device according to any one of Claims 1 to 9, **characterised in that** the ratio between the cross-section of the second tube (11) through which the solution passes and that of the first tube (8) through which the fluid passes is between 0.05 and 0.5, in particular equal to 0.14.

## Patentansprüche

1. Entnahmevorrichtung eines biologischen Mediums, insbesondere von Blut, mit Entnahmemitteln (1) des besagten Mediums, mindestens einem Beutel (2), der eine koagulationshemmende und/oder konservierende Lösung für das entnommene Medium enthält, und mindestens einem Sammelbeutel (3) für die Aufnahme des entnommenen, mit der koagulationshemmenden und/oder konservierenden Lösung versetzten Mediums, bei der der Sammelbeutel (3) über mindestens einen ersten flexiblen Stutzen (8) mit den Entnahmemitteln (1) und über mindestens einen zweiten flexiblen Stutzen (11) mit dem besagten Beutel (2) in flüssiger Verbindung steht, wobei die Vorrichtung ferner eine Schlauchquetschpumpe (13) umfasst, die jeweils den ersten (8) und den zweiten (11) Stutzen in einem so genannten Quetschbereich (14) teilweise einquetschen kann, wobei die Quetschgeschwindigkeit eines jeden Stutzens (8, 11) etwa identisch ist, und wobei das Verhältnis zwischen dem von der Lösung durchlaufenen Abschnitt des zweiten Stutzens (11) und dem von dem Medium durchlaufenen Abschnitt des ersten Stutzens (8) etwa dem dem entnommenen Medium hinzuzufügenden Volumenanteil der koagulationshemmenden und/oder konservierenden Lösung entspricht, so dass der Sammelbeutel (3) bei Betätigung der Pumpe (13) mit dem Medium und der koagulationshemmenden und/oder konservierenden Lösung in dem gewünschten Volumenanteil versorgt wird, wobei die besagte Vorrichtung **dadurch gekennzeichnet ist, dass** der erste (8) und der zweite (11) Stutzen vorgesehen sind, um das Mischen der koagulationshemmenden und/oder konservierenden Lösung mit dem Medium stromabwärts des Quetschbereichs (14) zu ermöglichen.

2. Vorrichtung nach Anspruch 1, **dadurch gekennzeichnet, dass** die Schlauchquetschpumpe (13) den ersten (8) und den zweiten (11) Stutzen gleichzeitig einquetschen kann, um den Sammelbeutel (3) gleichzeitig mit dem Medium und der koagulationshemmenden und/oder konservierenden Lösung zu versorgen.

3. Vorrichtung nach Anspruch 1 oder 2, **dadurch gekennzeichnet, dass** stromabwärts des Quetschbereichs (14) eine Verbindung (20) vorgesehen ist, um den ersten (8) und den zweiten (11) Stutzen an ein erstes Ende (21) eines Stutzens (22) anzuschließen, dessen zweites Ende (23) an einem Eingangsanschluss (5) des Sammelbeutels (3) angeschlossen ist.

4. Vorrichtung nach einem der Ansprüche 1 bis 3, **dadurch gekennzeichnet, dass** die Schlauchquetschpumpe (13) einen Motor (15) umfasst, der eine Welle (16) antreibt, an der zwei identische zylindrische Rotoren (17, 18) montiert sind, wobei die besagten Rotoren jeweils Rollen (19) tragen, die jeweils den ersten (8) und den zweiten (11) Stutzen einquetschen können.

5. Vorrichtung nach Anspruch 1 oder 2, **dadurch gekennzeichnet, dass** der erste (8) und der zweite (11) Stutzen stromaufwärts des Quetschbereichs (14) in einem Stutzen (24) dicht miteinander verbunden sind, dessen stromabwärtiges Ende (25) an einem Eingangsanschluss (5) des Sammelbeutels (3) angeschlossen ist.

6. Vorrichtung nach Anspruch 5, **dadurch gekennzeichnet, dass** der erste (8) und der zweite (11) Stutzen stromabwärts des Quetschbereichs (14) unterbrochen sind, so dass der Sammelbeutel (3) über den Stutzen (24) mit dem Medium und der koagulationshemmenden und/oder konservierenden Lösung versorgt wird.

7. Vorrichtung nach Anspruch 1 oder 2, **dadurch gekennzeichnet, dass** der zweite Stutzen (11) stromaufwärts des Quetschbereichs (14) dicht in den ersten Stutzen (8) eingeführt ist.

8. Vorrichtung nach Anspruch 7, **dadurch gekennzeichnet, dass** der zweite Stutzen (11) stromabwärts des Quetschbereichs (14) unterbrochen ist, so dass der Sammelbeutel (3) über den ersten Stutzen (8) mit dem Medium und der koagulationshemmenden und/oder konservierenden Lösung versorgt wird.

9. Vorrichtung nach einem der Ansprüche 5 bis 8, **dadurch gekennzeichnet, dass** die Schlauchquetschpumpe (13) einen Motor (15) umfasst, der eine Welle (16) antreibt, an der ein zylindrischer Rotor (17) montiert ist, wobei der besagte Rotor Rollen (19) trägt, die den ersten (8) und den zweiten (11) Stutzen einquetschen können.

10. Vorrichtung nach einem der Ansprüche 1 bis 9, **dadurch gekennzeichnet, dass** das Verhältnis zwischen dem von der Lösung durchlaufenen Abschnitt des zweiten Stutzens (11) und dem von dem Medium durchlaufenden Abschnitt des ersten Stutzens (8) zwischen 0,05 und 0,5 beträgt, insbesondere 0,14.
